(19) Europäisches Patentamt
European Patent Office
Office européen des brevets

(11) EP 0 585 157 B1

(12) **FASCICULE DE BREVET EUROPEEN**

(45) Date de publication et mention de la délivrance du brevet: **20.03.1996 Bulletin 1996/12**

(51) Int. Cl.$^6$: **C07C 323/12**, A61K 7/42, A61K 47/20, B01F 17/00

(21) Numéro de dépôt: **93401976.1**

(22) Date de dépôt: **29.07.1993**

(54) **Composés amphiphiles soufrés, fluorés et polyglycérolés, composition cosmétique ou pharmaceutique en comportant, procédé de préparation et vésicules formées**

Amphiphilische fluorierte Schwefel-Polyglycerol Verbindungen, ihre enthaltende kosmetische oder pharmazeutische Zusammensetzungen, Verfahren zu ihrer Herstellung und gestattende Bläschen

Amphiphilic fluorinated polyglycerol sulphur compounds cosmetic or pharmaceutical compositions containing them, process for preparation and formed vesicles

(84) Etats contractants désignés:
**AT BE CH DE DK ES GB GR IE IT LI NL PT SE**

(30) Priorité: **29.07.1992 FR 9209404**

(43) Date de publication de la demande: **02.03.1994 Bulletin 1994/09**

(73) Titulaire: **L'OREAL**
**F-75008 Paris (FR)**

(72) Inventeurs:
- **Bollens, Eric**
  **F-94410 Saint Maurice (FR)**
- **Mahieu, Claude**
  **F-75017 Paris (FR)**

(74) Mandataire: **Casalonga, Axel et al**
**BUREAU D.A. CASALONGA - JOSSE**
**Morassistrasse 8**
**D-80469 München (DE)**

(56) Documents cités:
**EP-A- 0 010 523**
**EP-A- 0 165 853**
**DE-A- 2 052 579**
**FR-A- 2 199 536**
**FR-A- 2 669 023**
**GB-A- 2 221 683**
**US-A- 3 759 874**
**US-A- 3 872 058**

Il est rappelé que: Dans un délai de neuf mois à compter de la date de publication de la mention de la délivrance du brevet européen, toute personne peut faire opposition au brevet européen délivré, auprès de l'Office européen des brevets. L'opposition doit être formée par écrit et motivée. Elle n'est réputée formée qu'après paiement de la taxe d'opposition. (Art. 99(1) Convention sur le brevet européen).

EP 0 585 157 B1

Printed by Rank Xerox (UK) Business Services
2.9.13/3.4

## Description

La présente invention concerne des composés soufrés, fluorés et polyglycérolés, utiles notamment dans des compositions cosmétiques ou pharmaceutiques, leur procédé de préparation ainsi que des compositions cosmétiques comportant ces composés, à titre de tensio-actifs, où certains de ces composés constituent des lipides amphiphiles non-ioniques capables de former des vésicules à structure lamellaire.

Dans le domaine de la cosmétique, notamment il est connu d'utiliser des perfluoropolyéthers dans des procédés de nettoyage, de protection, de maquillage de la peau ou bien de lavage des cheveux.

Le brevet français 2 669 023 décrit quelques composés amphiphiles non-ioniques dérivés du glycerol qui peuvent être utilisés comme agents tensio-actifs et comme agents émulsifiants.

La demanderesse a maintenant découvert de nouveaux composés qui présentent des propriétés tensio-actives, à titre d'émulsionnant, de dispersant ou de moussant et dont certains ont la capacité de former des vésicules présentant une bonne rémanence, contrairement à d'autres perfluorométhylisopropyléthers, notamment ceux qui sont connus sous le nom de "FOMBLIN".

Ainsi, la présente invention concerne les composés de formule :

$$R_f\text{---}(CH_2)_m\text{---}S\text{---}G_n\text{---}H \qquad (I)$$

dans laquelle :

$R_f$ désigne un radical alkyle perfluoré, linéaire ou ramifié, en $C_6$ à $C_{20}$ ou un mélange de radicaux alkyle perfluorés, linéaires ou ramifiés, en $C_4$ à $C_{20}$;

m représente O, 1 ou 2;

n représente une valeur statistique ou entière comprise entre 2 et 10;

G représente un motif choisi parmi :

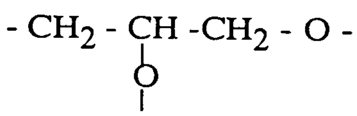 , 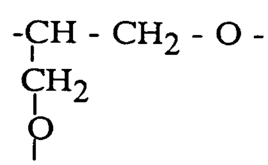 ,

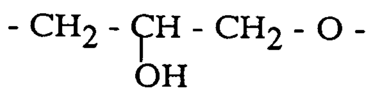 , 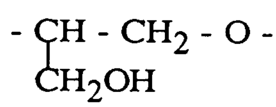 ,

ou

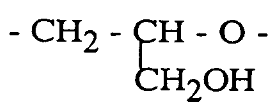 ,

chacun des atomes d'oxygène pouvant être relié à un atome d'hydrogène ou à un autre motif G.

Les composés préférés sont ceux pour lesquels $R_f$ désigne un radical alkyle perfluoré, linéaire ou ramifié, en $C_6$ à $C_{14}$ et m est 2.

Les composés de formule (I) selon l'invention peuvent être préparés en mettant en oeuvre la réaction d'un mercaptan fluoré de formule (II) :

$$R_f - (CH_2)_m\text{-}S\text{-}H \qquad (II)$$

dans laquelle $R_f$ et m ont les mêmes significations que dans la formule (I) en présence d'une quantité active de catalyseur basique,

a) par condensation avec n moles

- de glycidol, ou
- d'un composé à fonction époxyde susceptible, après réaction, de régénérer une fonction alcool,

éventuellement suivie d'une neutralisation;
ou
b) sur l'éther de glycidyle et d'isopropylidèneglycéryle, quand n, valeur entière, est égale à 2 ou un multiple de 2, éventuellement suivie d'une hydrolyse;
ou
c) sur l'éther de glycidyle et de diisopropylidènetriglycéryle, quand n est valeur entière égale à 4, éventuellement suivie d'une hydrolyse.

Le catalyseur basique utilisé peut être notamment choisi parmi les hydroxydes de métaux alcalins, les alcoolates de métaux alcalins tels que le méthylate de sodium, le tertiobutylate de potassium, les hydrures alcalins tels que l'hydrure de sodium, les amines tertiaires telles que la pyridine ou la triéthylamine. Ils peuvent aussi être des bases de LEWIS parmi lesquelles on peut citer les fluorures de césium, de rubidium, de potassium. Ces composés catalyseurs peuvent être également supportés sur un solide tel que l'alumine. De préférence, on utilise un alcoolate alcalin tel que le tertiobutylate de potassium ou le méthylate de sodium.

Selon l'invention, la concentration de catalyseur mis en oeuvre est comprise entre 1 et 20% molaire, de préférence 5 et 10% molaire par rapport au mercaptan fluoré de formule (II) utilisé.

Comme solvant pour la mise en oeuvre de la réaction, on peut choisir notamment parmi les hydrocarbures aromatiques tels que le toluène, le cumène, l'orthoxylène, le métaxylène, le paraxylène et leurs mélanges.

On peut mélanger d'abord le mercaptan fluoré de formule (II) avec le catalyseur basique. Pour réaliser le mélange, on peut opérer à une température comprise entre 40 et 160°C, de préférence entre 60 et 140°C. On peut opérer sous atmosphère inerte. Par atmosphère inerte, on entend un milieu réactionnel gazeux comportant essentiellement de l'azote, de l'argon, de l'hélium et leurs mélanges.

On ajoute alors le glycidol au mélange obtenu. L'addition peut s'effectuer en une seule fois ou progressivement, dans une période de temps pouvant aller de 30 minutes à 2 heures, par exemple.

A la fin de la réaction, il peut en outre être nécessaire de neutraliser le mélange obtenu à l'aide d'un acide minéral ou organique.

Comme cela est prévu ci-dessus, on peut préparer les mêmes composés en utilisant un composé à fonction époxyde susceptible, après réaction, de régénérer une fonction alcool et il peut s'agir notamment de l'épichlorhydrine, du t-butylglycidyléther ou du benzylglycidyl-éther.

Dans une seconde variante du procédé, pour le cas où n, est une valeur entière égale à 2 ou à un multiple de 2, les composés de formule (I) correspondants peuvent être préparés par réaction du mercaptan fluoré de formule (II) correspondant avec l'éther de glycidyle et d'isopropylidèneglycéryle, dans les mêmes conditions que celles qui sont décrites ci-dessus.

De même, dans une troisième variante, pour les composés où n est une valeur entière égale à 4, les composés de formule (I) correspondants peuvent être préparés par réaction équimolaire du mercaptan fluoré de formule (II) correspondant avec l'éther de glycidyle et de diisopropylidènetriglycéryle, dans les mêmes conditions que celles qui sont décrites ci-dessus.

Dans ces deux derniers cas, il est possible soit de purifier le composé intermédiaire non hydrolysé, qui subit dans un second temps une réaction d'hydrolyse, soit de ne pas le purifier, le produit étant alors purifié après hydrolyse du composé intermédiaire. La purification peut être par exemple réalisée par distillation.

La réaction d'hydrolyse peut être effectuée en présence d'acide minéral comme l'acide chlorhydrique, sulfurique ou phosphorique ou encore en présence d'un acide organique comme l'acide acétique, lactique ou méthanesulfonique. Le milieu réactionnel peut alors être neutralisé, si nécessaire, à l'aide d'une base comme la soude ou la potasse.

Les composés de formule (I) selon l'invention, peuvent se présenter sous forme d'huile ou sous forme de pâte ou de solide à la température ambiante. Ces composés sont solubles ou dispersibles dans l'eau. Ils présentent en outre des propriétés moussantes, émulsionnantes ou dispersantes.

Un autre objet de l'invention concerne l'utilisation des produits de formule (I), à titre d'agents tensio-actifs. Ils peuvent notamment être utilisés dans des compositions cosmétiques pour la peau et ses phanères ou dans des compositions pharmaceutiques ou dermopharmaceutiques.

Ainsi, l'invention concerne également les compositions cosmétiques ou pharmaceutiques comportant au moins un composé de formule (I) :

$$R_f - (CH_2)_m - S - G_n - H \qquad (I)$$

dans laquelle :
$R_f$ désigne un radical alkyle perfluoré, linéaire ou ramifié, en $C_6$ à $C_{20}$ ou un mélange de radicaux alkyle perfluorés, linéaires ou ramifiés, en $C_4$ à $C_{20}$;
m représente O, 1 ou 2;
n représente une valeur statistique ou entière comprise entre 2 et 10;
G représente un motif choisi parmi :

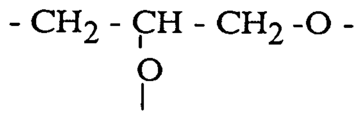
,
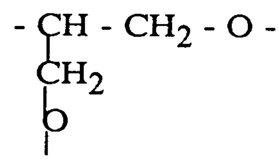
,

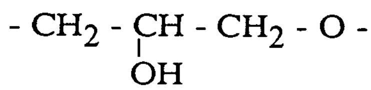
,
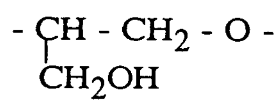
,

ou

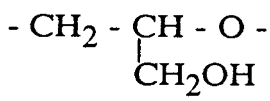
,

chacun des atomes d'oxygène pouvant être relié à un atome d'hydrogène ou à un autre motif G.

Ces compositions peuvent se présenter sous forme d'émulsions, de microémulsions, de laits ou de crèmes, de lotions, de gels, de bâtonnets solides, de pâtes, de sprays ou de mousses aérosols.

Dans ces compositions, les composés de formule (I) représentent de 0,1 à 50%, et de préférence de 0,1 à 25% en poids du poids total de la composition.

Dans les compositions selon l'invention, les composés de formule (I) peuvent être associés à d'autres agents de surface ioniques ou non-ioniques, à des polymères naturels ou synthétiques, ioniques ou non-ioniques, à des huiles ou des cires, à des protéines plus ou moins hydrolysées, à des épaississants, à des nacrants, à des émollients, à des hydratants, à des colorants, à des agents réducteurs ou oxydants, à des conservateurs, à des parfums, à des filtres anti-UV, à des solvants, à des propulseurs ou à des produits actifs pharmaceutiques ou parapharmaceutiques.

Parmi les huiles, on peut citer, de façon non limitative, les huiles minérales telles que la vaseline, les huiles animales telles que les huiles de baleine, de phoque, de foie de flétan, de foie de morue, de thon, de vison, les huiles végétales telles que les huiles d'amande, d'arachide, de germe de blé, de maïs, d'olive, de jojoba, de sésame et de tournesol, les huiles de silicone, et les huiles partiellement ou entièrement perfluorées telles que la perfluorodécaline, les perfluoro alkyl bromures tels que le bromure de perfluorooctyle, les perfluorométhylisopropyléthers connus sous le nom de "FOMBLIN HC" commercialisés par la Société MONTEFLUO.

Parmi les cires, on peut citer, de façon non limitative, la cire de sipol, la lanoline, la lanoline hydrogénée, la lanoline acétylée, la cire d'abeille, la cire de candelila, la cire microcristalline, la paraffine, la cire de carnauba, le spermaceti, le beurre de cacao, le beurre de karité, les cires de silicone et les huiles hydrogénées concrètes à 25°C. Les huiles et les cires peuvent également être choisies parmi les esters d'acides gras en $C_{12}$ à $C_{22}$, saturés ou insaturés et d'alcools ou polyols inférieurs comme l'isopropanol, le glycol ou le glycérol, ou d'alcools gras en $C_8$-$C_{22}$, linéaires ou ramifiés, saturés ou insaturés, ou encore d'alcane diols-1,2 en $C_{10}$-$C_{22}$.

Lorsque les compositions se présentent sous forme d'émulsion de type eau-dans-huile ou huile-dans-eau, les composés de formule (I) peuvent être associés à d'autres agents émulsionnants classiques tels que des acides gras ou des alcools gras polyoxyéthylénés, des alkyléthers de polyglycérol, des esters d'acide gras et de sorbitan polyoxyéthylénés ou non, des esters d'acide gras et de sorbitol polyoxyéthylénés ou non, l'huile de ricin polyoxyéthylénée, des sels d'acides gras et d'amines ou de métaux polyvalents, des alkylsulfates polyoxyéthylénés ou non et des alkylphosphates polyoxyéthylénés ou non.

Les compositions cosmétiques ou pharmaceutiques selon l'invention peuvent se présenter sous forme de shampooings ou d'après-shampooings, de bains moussants, de compositions nettoyantes, de bains ou de crèmes pour le soin de la peau et des cheveux, de compositions anti-solaires, de crèmes ou mousses de rasage, de lotions après-rasage, de déodorants corporels, de compositions à usage bucco-dentaire, des compositions tinctoriales capillaires, de compositions de coloration de la peau ou encore de compositions de maquillage, par exemple.

La demanderesse a mis en évidence, en outre, que certains composés de formule (I) et en particulier les composés de formule (I) pour lesquels $R_f$ représente un radical alkyle perfluoré linéaire en $C_6$ à $C_{10}$, m est égal à 2 et n représente une valeur statistique ou entière comprise entre 2 et 3, sont des lipides amphiphiles non-ioniques capables de former des vésicules à structure lamellaire.

De façon connue, ces vésicules sont caractérisées par une structure en feuillets constitués de couches de phase lipidique encapsulant une phase aqueuse.

Ces vésicules sont, de façon connue, préparées sous forme de dispersion dans une phase aqueuse. On trouvera une liste non limitative de divers modes de préparation dans *"Les liposomes en biologie cellulaire et pharmacologie"* - Editions INSERM - John LIBLEY, Eurotext, 1987, pages 6 à 18.

Les vésicules obtenues sont donc constituées d'une phase lipidique constituée d'un ou plusieurs feuillets encapsulant une phase E aqueuse et elles sont dispersées dans une phase aqueuse de dispersion D.

Les vésicules formées avec les composés de formule (I) pour lesquels $R_f$ représente un radical alkyle perfluoré linéaire en $C_6$-$C_{10}$, m est égal à 2 et n représente une valeur statistique ou entière comprise entre 2 et 3, possèdent dans l'ensemble un bon taux de gonflement, une faible perméabilité et une bonne stabilité.

La présente invention a également pour objet les vésicules à structure lamellaire, délimitées par un ou plusieurs feuillets formés d'une phase lipidique encapsulant une phase aqueuse et contenant au moins un composé de formule (I), pour lesquels $R_f$ représente un radical alkyle perfluoré linéaire en $C_6$-$C_{10}$, m est égal à 2 et n est une valeur statistique ou entière, comprise entre 2 et 3.

De façon surprenante, on a constaté qu'une composition, contenant une dispersion vésiculaire préparée avec les composés selon l'invention, présente une bonne rémanence à l'eau après application sur la peau. Une application cosmétique préférée de ces dispersions vésiculaires concerne donc les produits de coloration artificielle de la peau et les produits antisolaires.

La présente invention a donc également pour objet une composition cosmétique ou pharmaceutique contenant, dispersées dans une phase aqueuse de dispersion D, des vésicules délimitées par un ou plusieurs feuillets formés d'une phase lipidique contenant au moins un composé de formule (I) dans laquelle $R_f$ représente un radical alkyle perfluoré linéaire en $C_6$-$C_{10}$, m est égal à 2 et n représente une valeur statistique ou entière comprise entre 2 et 3.

Selon l'invention, tous les lipides amphiphiles ioniques et/ou non-ioniques susceptibles de former des vésicules stables, seuls ou en mélange avec des additifs ayant pour fonction de diminuer la perméabilité des membranes des vésicules et d'améliorer leur stabilité, peuvent être utilisés en mélange avec les composés de formule (I) pour constituer les membranes lipidiques des vésicules selon l'invention. La phase lipidique constitutive des membranes des vésicules de la dispersion selon l'invention peut donc comprendre, de façon connue, au moins un lipide choisi dans le groupe formé par:

**A/** les lipides non-ioniques ci-après définis :

(1) les dérivés du glycérol, linéaires ou ramifiés, de formule :

$$R_oO - [C_3H_5(OH)O]_q - H \qquad (II)$$

dans laquelle :

- $-C_3H_5(OH)O-$
  est représenté par les structures suivantes, prises en mélange ou séparément :
  $-CH_2CHOHCH_2O-$,

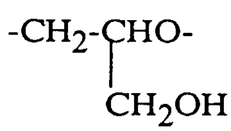 ou 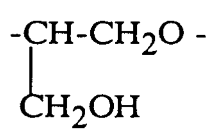

- q est une valeur statistique moyenne comprise entre 1 et 6 ou bien q = 1 ou 2, auquel cas $-C_3H_5(OH)O-$ est représenté par la structure $-CH_2-CHOH-CH_2O$;
- $R_o$ représente :

  (a) une chaîne aliphatique, linéaire ou ramifiée, saturée ou insaturée, contenant de 12 à 30 atomes de carbone; ou des radicaux hydrocarbonés des alcools de lanoline; ou les restes d'alpha-diols à longue chaîne;

(b) un reste $R_1CO$, où $R_1$ est un radical aliphatique, linéaire ou ramifié, en $C_{11}$-$C_{29}$ ;

(c) un reste

$R_2$-[$OC_2H_3(R_3)$]-

où :

. $R_2$ peut prendre la signification (a) ou (b) donnée pour $R_o$;

. -$OC_2H_3(R_3)$- est représenté par les structures suivantes, prises en mélange ou séparément :

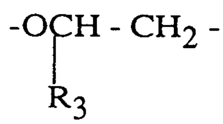

et

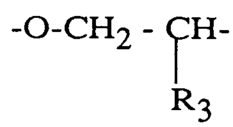

où $R_3$ prend la signification (a) donnée pour $R_o$;

(2) les éthers de polyglycérol, linéaires ou ramifiés, comportant deux chaînes grasses ;

(3) les diols à chaîne grasse ;

(4) les alcools gras, oxyéthylénés ou non, les stérols, tels que le b-sitostérol, le cholestérol et les phytostérols, oxyéthylénés ou non ;

(5) les éthers et esters de polyols, oxyéthylénés ou non, l'enchaînement des oxydes d'éthylène pouvant être linéaire ou cyclique ;

(6) les glycolipides d'origine naturelle ou synthétique, les éthers et esters de mono- ou polysaccharides et, notamment, les éthers et les esters de glucose ;

(7) les hydroxylamides représentés par la formule :

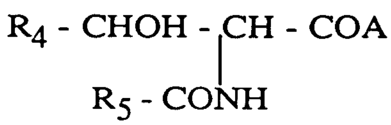

(III)

dans laquelle :

- $R_4$ désigne un radical alkyle ou alcényle en $C_7$-$C_{21}$ ;
- $R_5$ désigne un radical hydrocarboné, saturé ou insaturé, en $C_7$-$C_{31}$ ;
- COA désigne un groupement choisi parmi les deux groupements suivants :

. un reste

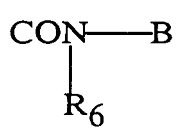

où :

. B est un radical alcoyle dérivé d'amines primaires ou secondaires, mono- ou polyhydroxylées ; et

. $R_6$ désigne un atome d'hydrogène ou un radical méthyle, éthyle ou hydroxyéthyle ; et

. un reste -COOZ, où Z représente le reste d'un polyol en $C_3$-$C_7$.

(8) les céramides naturels ou de synthèse ;

(9) les dihydroxyalkylamines, les amines grasses oxyéthylénées ;

(10) les dérivés du glycérol décrits dans la demande de brevet PCT n° 92/08685 et répondant à la formule :

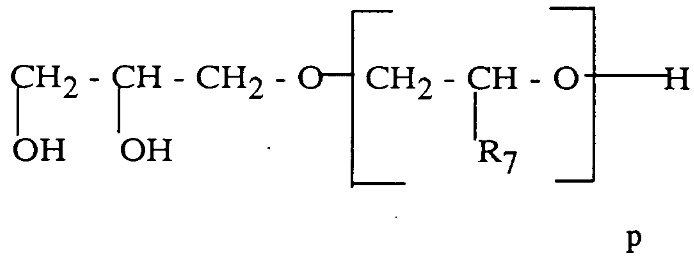

(IV)

dans laquelle $R_7$ représente un radical alkyle linéaire en $C_{14}$ à $C_{18}$ ou un groupement $-CH_2Y$ dans lequel Y est $-OR_8$, $R_8$ représentant un radical alkyle linéaire en $C_{10}$-$C_{18}$ et, de préférence, en $C_{16}$, et p représente une valeur statistique moyenne supérieure à 1 et au plus égale à 3 et, en outre, lorsque $R_7$ est $-CH_2Y$, p peut également représenter une valeur entière égale à 2.

**B/** les lipides amphiphiles ioniques ci-après définis :

(1) les lipides amphiphiles anioniques suivants :

. les phospholipides naturels, les phospholipides modifiés par voie chimique ou enzymatique, et les phospholipides de synthèse ;
. les composés anioniques de formule :

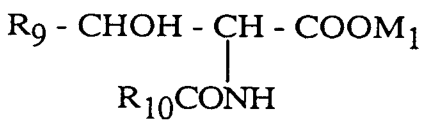

(V)

dans laquelle :

- $R_9$ représente un radical alkyle ou alcényle en $C_7$-$C_{21}$ ;
- $R_{10}$ représente un radical hydrocarboné, saturé ou insaturé, en $C_7$-$C_{31}$, et
- $M_1$ représente H, Na, K, $NH_4$ ou un ion ammonium substitué dérivé d'une amine ;

. les composés anioniques, tels que les esters phosphoriques d'alcools gras, notamment le dicétylphosphate et le dimyristylphosphate sous forme d'acides ou de sels alcalins ; l'acide heptylnonylbenzène sulfonique ; le sulfate de cholestérol acide ou le phosphate de cholestérol acide, ainsi que leurs sels alcalins ; les lysolécithines ; les alkylsulfates tels que le cétylsulfate de sodium ; les gangliosides ;
(2) les lipides amphiphiles cationiques suivants :

. les composés cationiques ayant la formule :

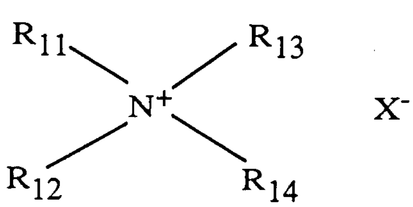

(VI)

dans laquelle $R_{11}$ et $R_{12}$, identiques ou différents, représentent des radicaux alkyle en $C_{12}$-$C_{20}$ et $R_{13}$ et $R_{14}$, identiques ou différents, des radicaux alkyle en $C_1$-$C_4$ ;
. les amines à longue chaîne et leurs dérivés ammonium quaternaire ; les esters d'aminoalcools à longue chaîne et leurs sels et dérivés ammonium quaternaire ; et

. les lipides polymérisables, comme décrits par RINGSDORF et autres, dans "Angewandte Chemie.", vol. 27, n° 1, Janvier 1988, pages 129-137.

On peut aussi ajouter à la phase lipidique constituant les parois vésiculaires des additifs, tels que certains polymères comme, par exemple, les polypeptides et les protéines.

La phase aqueuse de dispersion selon l'invention peut être constituée par de l'eau, ou un mélange d'eau et d'au moins un solvant miscible à l'eau tels que les alcools en $C_1$-$C_7$ et les polyols d'alkyle en $C_1$-$C_5$. La phase aqueuse peut également contenir des composés en solution, tels que des sucres, des sels organiques ou minéraux ou des polymères.

La phase aqueuse de dispersion peut aussi contenir une dispersion de gouttelettes d'un liquide non miscible à l'eau que les vésicules stabilisent, de sorte qu'il n'est pas nécessaire d'introduire pour cette stabilisation un émulsionnant, tel que le monostéarate de glycérol par exemple ; ce liquide non miscible à l'eau peut être choisi dans le groupe formé par les huiles animales ou végétales, les huiles essentielles naturelles ou synthétiques, les hydrocarbures, les carbures halogénés, les silicones, les esters d'un acide minéral et d'un alcool, les éthers et les polyéthers. Des exemples de liquides non miscibles à l'eau sont mentionnés dans le brevet européen 455 528.

Dans la composition selon l'invention, la phase lipidique totale de la dispersion représente avantageusement entre 0,01 % et 50% en poids et, de préférence, entre 1 et 20% en poids, par rapport au poids total de la dispersion. Le (ou les) lipide(s) amphiphile(s) représente(nt) avantageusement entre 10 et 95% en poids et, de préférence, de 40 à 90% en poids par rapport au poids total de la phase lipidique vésiculaire. Les vésicules ont, de préférence, des dimensions comprises entre 20 et 3 000 nanomètres, plus particulièrement entre 20 et 500 nanomètres.

Les compositions obtenues avec les composés selon l'invention peuvent contenir, de façon connue, un ou plusieurs composé(s) actif(s) ayant une activité cosmétique et/ou dermopharmaceutique, qui, selon leurs caractéristiques de solubilité, peuvent avoir différentes localisations. Par exemple, dans le cas des dispersions de vésicules contenant une phase aqueuse encapsulée, si les actifs sont liposolubles, on les introduit dans la phase lipidique constituant le(s) feuillet(s) des vésicules ou dans les gouttelettes de liquide non miscible à l'eau stabilisées par les vésicules; si les actifs sont hydrosolubles, on les introduit dans la phase aqueuse encapsulée des vésicules ou dans la phase aqueuse de dispersion; si les actifs sont amphiphiles, ils se répartissent entre la phase lipidique et la phase aqueuse encapsulée avec un coefficient de partage, qui varie selon la nature de l'actif amphiphile et les compositions respectives de la phase lipidique et de la phase aqueuse encapsulée. De façon générale, les actifs sont mis en place dans la phase lipidique des feuillets et/ou dans la phase encapsulée par les feuillets.

Dans le cas des émulsions de type huile-dans-eau ou eau-dans-huile contenant un composé de formule (I) comme agent émulsionnant, les composés liposolubles sont introduits dans la phase huileuse et les composés hydrosolubles dans la phase aqueuse. De même, les actifs amphiphiles se répartissent entre la phase aqueuse et la phase huileuse.

Comme actifs cosmétiques et/ou dermopharmaceutiques, on peut citer les anti-oxydants ou les anti-radicaux libres, les agents hydratants ou humectants, les agents mélanorégulateurs accélérateurs de bronzage, les agents mélanorégulateurs dépigmentants, les agents de coloration de la peau, les liporégulateurs, les agents anti-vieillissement et anti-rides, les agents anti-UV, les agents kératolytiques, les émollients, les agents anti-inflammatoires, les agents rafraîchissants, les agents cicatrisants, les agents protecteurs vasculaires, les agents anti-bactériens, les agents antifongiques, les agents insectifuges, les agents anti-perspirants, les agents déodorants, les agents anti-pelliculaires, les anti-chutes des cheveux, les colorants capillaires, les agents décolorants pour cheveux, les réducteurs pour permanente, les agents conditionneurs pour la peau et les cheveux. Ces actifs cosmétiques et/ou dermopharmaceutiques sont cités de façon plus détaillée dans la demande de brevet PCT n° 92/08685.

Les compositions selon l'invention peuvent aussi contenir, de façon connue, des additifs de formulation n'ayant ni activité cosmétique, ni activité dermopharmaceutique propre, mais utiles pour la formulation des compositions sous forme de lotion, crème ou sérum. Ces additifs sont, en particulier, pris dans le groupe formé par les gélifiants, les polymères, les conservateurs, les colorants, les opacifiants et les parfums. Parmi les gélifiants utilisables, on peut citer les dérivés de cellulose tels que l'hydroxyéthylcellulose, les dérivés d'algues tels que le satiagum, des gommes naturelles, telles que l'adragante, et des polymères synthétiques, en particulier les mélanges d'acides polycarboxyvinyliques commercialisés sous la dénomination commerciale "CARBOPOL" par la Société GOODRICH. Ces additifs sont plus particulièrement ajoutés dans une phase aqueuse, par exemple la phase aqueuse de dispersion de vésicules ou la phase aqueuse d'une émulsion huile-dans-eau ou eau-dans-huile.

Les exemples donnés ci-après, à titre illustratif, permettront de mieux comprendre l'invention.

## EXEMPLES DE PREPARATION

### EXEMPLE 1

**Composé de formule (I) pour lequel $\bar{n}$ = 3, $R_f = C_8F_{17}$ et m = 2**

Dans un réacteur sous atmosphère inerte sont introduits 108 g (0,225 mole) de 2-F-octyléthanethiol. Sous agitation et à la température de 25°C, on ajoute 1,26 g de tertiobutylate de potassium (11,25 meq). La température est amenée entre 50 et 60°C et le montage est placé sous un vide de 4000 Pa afin d'éliminer le tertiobutanol. La montée en température est poursuivie. Lorsque l'on atteint 80°C, on additionne en 30 minutes 16,65 g de glycidol (0,225 mole) en maintenant une température voisine de 80°C. Le milieu réactionnel devient pâteux. Afin de fluidifier le mélange, 59 g de xylène sont ajoutés en 15 minutes. Le chauffage est repris jusqu'à ce que la température atteigne 130°C.

A cette température, on ajoute en 75 minutes 33,3 g de glycidol (0,45 mole) goutte à goutte. A la fin de l'addition, la température est maintenue à 130°C pendant 15 minutes. La montée en température est reprise jusqu'à 155°C et le xylène est éliminé par distillation à pression atmosphérique.

Après 15 minutes à 155°C, le milieu est neutralisé par 11,5 ml de HCl 1N. Quelques mousses se forment puis le mélange devient translucide, la température a chuté à 135°C. A cette température, le produit est laissé environ 15 minutes sous 4000 Pa puis se solidifie à la température ambiante.

On obtient 157 g de produit.

Point de fusion : 64°C

| ANALYSE ELEMENTAIRE | | | | |
|---|---|---|---|---|
| | %C | %H | %S | %F |
| Calculé | 32,48 | 3,27 | 4,56 | 46,01 |
| Trouvé | 32,47 | 3,27 | 4,28 | 46,16 |

### EXEMPLE 2

**Composé de formule (I) dans lequel $\bar{n}$ = 2,5 et $R_f = C_8F_{17}$ et m = 2**

Selon un procédé équivalent à celui de l'exemple 1, le composé de l'exemple 2 est préparé par condensation de 108 g de 2-F-octyléthane thiol avec 41,63 g de glycidol. On obtient 150 g de produit.

Point de fusion : 71°C

| ANALYSE ELEMENTAIRE | | | | |
|---|---|---|---|---|
| | %C | %H | %S | %F |
| Calculé | 31,58 | 3,01 | 4,81 | 48,57 |
| Trouvé | 31,62 | 3,22 | 4,60 | 48,61 |

### EXEMPLE 3

**Composé de formule (I) pour lequel n = 2, $R_f = C_8F_{17}$ et m = 2 1-(F-octyl)5,9,10-trihydroxy 7-oxa 3-thiadécane**

**a)** ***Préparation du (2'-F-octyléthylthio)3-(isopropylidène glycéryl) 2-propanol***

Dans un réacteur, on introduit 192 g de 2-F-octyléthanethiol (0,4 mole) à la température de 25°C; on ajoute sous atmosphère inerte 3,61 g (20 meq) d'une solution de méthylate de sodium en solution dans le méthanol (titre : 5,54 meq/g). Le mélange est chauffé sous un vide de 4000 Pa de façon à éliminer le méthanol.

Le mélange réactionnel présente alors un aspect laiteux. La température dans le ballon à la fin de l'évaporation du méthanol est de 68-70°C. Le glycidyléther de l'isopropylidène glycérol (75,2 g - 0,4 mole) est ensuite additionné goutte-à-goutte en 1 heure après avoir arrêté le chauffage. L'exothermicité maintient la température entre 65 et 68°C. Le mélange est encore agité deux heures à la température de 25°C.

Le produit est purifié par distillation : Eb = 159°C/66,6 Pa.

| ANALYSE ELEMENTAIRE | | | | |
|---|---|---|---|---|
| | %C | %H | %S | %F |
| Calculé | 34,14 | 3,17 | 4,80 | 48,32 |
| Trouvé | 34,11 | 3,22 | 4,58 | 48,07 |

**b)** ***Hydrolyse du (2'-F-octyléthylthio)3-(isopropylidène glycéryl) 2-propanol***

Dans un réacteur, on introduit 85 g (0,127 mole) du produit obtenu à l'exemple 3a) en solution dans 65 ml d'isopropanol. A la température de 25°C, on ajoute goutte-à-goutte en 15 minutes 3,5 ml d'acide chlorhydrique concentré (33%).

Le mélange est porté à la température de 60°C pendant 7 heures.

Le milieu est alors neutralisé avec une solution de soude à 10%. Après évaporation des solvants et retour à température ambiante, le mélange est repris dans l'isopropanol puis filtré. Après évaporation du filtrat, on obtient 68 g d'un solide blanc collant.

Point de fusion : 87°C

| ANALYSE ELEMENTAIRE | | | | |
|---|---|---|---|---|
| | %C | %H | %S | %F |
| Calculé | 30,57 | 2,70 | 5,10 | 51,43 |
| Trouvé | 30,53 | 2,76 | 4,96 | 51,62 |

EXEMPLE 4

**Composé de formule (I) pour lequel n = 2, $R_f$ = $C_6F_{13}$ et m = 2 1-(F-hexyl)5,9,10-trihydroxy 7-oxa 3-thiadécane**

**a)** ***Préparation du 1-(2'-F-hexyléthylthio)3-(isopropylidène glycéryl) 2-propanol***

Selon le mode opératoire décrit à l'exemple 3a), le composé est préparé par réaction de 152 g de 2-F-hexyléthanethiol avec 75,2 g d'éther de glycidyle et d'isopropylidèneglycéryle.

Le produit est purifié par distillation : Eb = 140°C/67 Pa.

On obtient 112 g d'un liquide transparent.

| ANALYSE ELEMENTAIRE | | | | |
|---|---|---|---|---|
| | %C | %H | %S | %F |
| Calculé | 35,92 | 3,72 | 5,64 | 43,45 |
| Trouvé | 35,06 | 3,60 | 5,40 | 43,01 |

**b)** ***Hydrolyse du 1-(2'-F-hexyiéthylthio)3-(isopropylidène glycéryl) 2-propanol***

Dans un réacteur de 250 ml, on introduit 85 g (0,15 mole) du produit obtenu à l'exemple 4a), en solution dans 65 ml d'isopropanol. A 25°C, on ajoute 7,2 ml d'acide chlorhydrique concentré en 15 minutes.

Le mélange est chauffé à 60°C pendant 7 heures. Le milieu est alors neutralisé par une solution de soude à 10%. Après évaporation des solvants et retour à la température de 25°C, le mélange est repris dans l'isopropanol puis filtré. Après évaporation du filtrat, on obtient 78 g d'un produit amorphe, translucide.

Point de fusion : 65°C

| ANALYSE ELEMENTAIRE | | | | |
|---|---|---|---|---|
| | %C | %H | %S | %F |
| Calculé | 31,83 | 3,24 | 6,07 | 46,75 |
| Trouvé | 31,63 | 3,48 | 5,74 | 46,41 |

EXEMPLE 5

**Composé de formule (I) dans lequel n = 4, et $R_f = C_8F_{17}$ et m = 2 1-[(1',3'-diglycéryl)-2'-glycéryl]-3-(2"-F-octyléthylthio)glycérol**

Dans un réacteur de 500 ml, on introduit 192 g (0,4 mole) de 2-F-octyléthanethiol à 25°C et on ajoute sous atmosphère d'azote 2,24 g (20 meq) de tertiobutylate de potassium. Le mélange est porté à 60°C et le tertiobutanol formé est évaporé sous un vide de 2000 Pa pendant 10 minutes. A cette température sont ajoutés en une heure, 150,4 g (0,4 mole) d'éther de glycidyle et de diisopropylidène triglycéryle.

Après retour à 25°C, le mélange est hydrolysé par une solution de 40 ml d'acide chlorhydrique concentré dans 200 ml d'isopropanol. Cette solution est ajoutée en 10 minutes au mélange réactionnel qui est chauffé à 50°C pendant trois heures.

Après retour à 25°C, la solution est neutralisée avec une solution 12 N de NaOH.

Le mélange est alors filtré, le filtrat ambré est concentré sous vide. La masse visqueuse ainsi obtenue, reprise dans 200 ml d'isopropanol, est de nouveau filtrée.

Après évaporation sous vide du solvant, le résidu est purifié sur silice Merck 60H (éluant $CH_2Cl_2/CH_3OH$ : 90/10) pour conduire à 150 g de produit sous forme d'huile.

| ANALYSE ELEMENTAIRE | | | | |
|---|---|---|---|---|
| | %C | %H | %S | %F |
| Calculé | 34,03 | 3,76 | 4,13 | 41,49 |
| Trouvé | 33,85 | 3,72 | 4,22 | 41,36 |

EXEMPLE 6

**Composé de formule (I) dans lequel $\overline{n}$ = 5, $R_f = C_8F_{17}$ et m = 2**

Selon le même procédé que décrit à l'exemple 1, on condense 72 g (0,15 mole) de 2-F-octyléthanethiol avec 11,1 g (0,15 mole) de glycidol, en présence de 0,85 g de tert-butylate de potassium à 80°C. Puis, après avoir ajouté 60 g de xylène, on additionne à 130°C 44,4 g (0,6 mole) de glycidol.

En fin de réaction, le mélange est neutralisé par 7,5 ml de HCl normal.

On obtient 125 g d'un produit sous forme de pâte dont le point de fusion est d'environ 47,5°C.

| ANALYSE ELEMENTAIRE | | | | |
|---|---|---|---|---|
| | %C | %H | %S | %F |
| Calculé | 35,30 | 4,15 | 3,77 | 37,97 |
| Trouvé | 35,12 | 4,41 | 3,65 | 37,28 |

EXEMPLE 7

**Composé de formule (I) dans lequel $\overline{n} = 5$, $R_f = C_6F_{13}$ et m = 2**

Dans les conditions décrites à l'exemple 1, on condense 57 g (0,15 mole) de 2-F-hexyléthanethiol avec 11,1 g (0,15 mole) de glycidol en présence de 0,85 g (7,5 meq) de tertiobutylate de potassium à 80°C. Puis, après avoir ajouté 60 g de xylène, on additionne à 130°C 44,4 g (0,6 mole) de glycidol. Après évaporation du solvant et neutralisation, le produit est séché à 155°C.

On obtient 112 g d'un solide sous forme de pâte dont le point de fusion est d'environ 43,4°C.

| ANALYSE ELEMENTAIRE | | | | |
|---|---|---|---|---|
| | %C | %H | %S | %F |
| Calculé | 36,80 | 4,70 | 4,27 | 32,91 |
| Trouvé | 36,86 | 4,97 | 4,09 | 32,89 |

EXEMPLE 8

**Composé de formule (I) dans lequel $\overline{n} = 10$, $R_f = C_6F_{13}$ et m = 2**

Dans un réacteur placé sous atmosphère inerte, on introduit 135,7 g (3,57 mole) de 2-F-hexyléthanethiol. Sous agitation et à température ambiante (25°C), on ajoute 2 g de tertiobutylate de potassium (17,85 meq). La température est amenée entre 50 et 60°C et le montage est placé sous un vide de 2666 Pa pour éliminer le tertiobutanol formé. On ajoute à cette température 150 g de xylène. La montée en température est poursuivie. Lorsque l'on atteint 80°C, on additionne 26,4 g (0,357 mole) de glycidol en 15 minutes. La température est amenée à 130°C en 60 minutes. A cette température, on ajoute 237,9 g de glycidol en 30 minutes. A la fin de l'addition, on maintient le mélange à 130°C pendant 15 minutes.

La montée en température est reprise jusqu'à 155°C puis le xylène est éliminé par distillation d'abord à pression atmosphérique puis sous pression réduite à 2666 Pa. Après 20 minutes à 155°C, on neutralise avec 18 ml d'acide chlorhydrique normal.

Le produit est laissé 15 minutes sous 2666 Pa à 135°C. Après refroidissement à 25°C, on obtient 395 g de produit se présentant sous la forme d'une huile ambrée très visqueuse.

| ANALYSE ELEMENTAIRE | | | | |
|---|---|---|---|---|
| | %C | %H | %S | %F |
| Calculé | 40,72 | 5,84 | 2,86 | 22,03 |
| Trouvé | 40,24 | 6,53 | 2,67 | 21,78 |

Dans les exemples 1, 2, 6, 7, et 8, $\overline{n}$ représente une valeur statistique et est déterminée par le rapport molaire glycidol sur mercaptan.

**EXEMPLES DE FORMULATION**

EXEMPLE 1

**CREME COLOREE**

Dans un bécher en verre, on pèse les produits suivants :

| | |
|---|---|
| - Composé de l'exemple 1 | 14 g |
| - Cholestérol | 4 g |
| - Dicétyl phosphate | 2 g |

On réalise le mélange de ces trois produits par fusion à la température de 100°C sous atmosphère d'azote. Puis on ramène la température du mélange fondu à 90°C. On ajoute alors 20 g d'eau contenant 7 g de caramel commercialisé par la Société CERESTAR sous la dénomination de "CERESTAR CARAMEL 15610", et on homogénéise le mélange obtenu à la température de 90°C.

On ajoute alors 34,65 g d'eau contenant 13 g de caramel.

A la température de 70°C, on homogénéise le mélange à l'aide d'un ultra disperseur du type Virtis pendant 4 minutes à la vitesse de 40.000 t/mn.

On ajoute, enfin, 5 g d'eau contenant 0,3 g de conservateur constitué de diazolidinyl-urée vendue sous la dénomination "GERMAL II" par la Société SUTTON et 0,05 g de conservateur constitué par un mélange de méthylchloroisothiazolinone et de méthylisothiazolinone vendu sous la dénomination "KATHON CG" par la Société ROHM & HAAS.

On obtient ainsi une dispersion de vésicules épaisses, de couleur brune.

EXEMPLE 2

**CREME DE BRONZAGE ARTIFICIEL**

Selon le mode opératoire de l'exemple 1, on réalise :

***1ère phase*** :

| | |
|---|---|
| - Composé de l'exemple 1 | 6 g |
| - Cholestérol | 1,6g |
| - Stéaroylglutamate monosodique vendu sous la dénomination "ACYL GLUTAMATE HS11" par la Société AJINOMOTO | 0,4g |
| - Dihydroxyacétone | 3 g |
| - Caramel vendu sous la dénomination "CERESTAR CARAMEL 15610" par la Société CERESTAR | 3 g |
| - EAU | 40 g |
| - Mélange de méthylchloroisothiazolinone et de méthylisothiazolinone vendu sous la dénomination "KATHON CG" par la Sociét ROHM & HAAS | 0,1g |
| - Diazolidinylurée vendue sous la dénomination "GERMAL II" par la Société SUTTON | 0,3g |
| - Eau | 5 g |

*__2ème phase__* :

On ajoute à la phase vésiculaire obtenue ci-dessus :

| | |
|---|---|
| - Huile de macadamia | 15 g |
| - Huile de silicone volatile | 10 g |

A la température de 30°C, on homogénéise le tout à l'aide d'un ultra disperseur Virtis pendant 4 minutes à la vitesse de 40.000 t/mn.

On ajoute enfin :

| | |
|---|---|
| - Ether de l'alcool cétylique et de l'hydroxyéthylcellulose vendu sous la dénomination "NATROSOL PLUS CS" par la Société AQUALON | 0,7 g |
| - Paraoxybenzoate de méthyle | 0,2 g |
| - Eau | qsp 100 g |

On obtient ainsi une crème épaisse.

EXEMPLE 3

**LAIT DE SOIN ANTISOLAIRE**

Selon le mode opératoire de l'exemple 2, on prépare le produit de composition :

| | |
|---|---|
| - Composé de l'exemple 1 | 4,5 g |
| - Cholestérol | 1,2 g |
| - Dicétyl phosphate | 0,3 g |
| - Acétate de vitamine E | 0,3 g |
| - Glycérine | 3 g |
| - Eau | 40 g |
| - Mélange de méthylchloroisothiazolinone et de méthylisothiazolinone vendu sous la dénomination "KATHON CG" par la Société ROHM & HAAS | 0,1 g |
| - Diazolidinylurée vendue sous la dénomination "GERMAL II" par la Société SUTTON | 0,3 g |
| - Eau | 5 g |
| - Huile de vaseline | 15 g |
| - Paradiméthylaminobenzoate de 2-éthyl hexyle | 3 g |
| - Acide polyacrylique réticulé vendu sous la dénomination "CARBOPOL 940" par la Société GOODRICH | 0,42 g |
| - Paraoxybenzoate de méthyle | 0,2 g |
| - Triéthanolamine | 0,4 g |
| - Eau | qsp 100 g |

EXEMPLE 4

**SERUM DE SOIN VITAMINE ANTI-AGE**

Selon le mode opératoire de l'exemple 2, on prépare le produit de composition :

| | | |
|---|---|---|
| - Composé de l'exemple 4 | | 1,8 g |
| - Cholestérol | | 0,6 g |
| - Cholestérol sulfate de sodium | | 0,6 g |
| - Acétate de vitamine E | | 0,15 g |
| - Palmitate de vitamine A | | 0,15 g |
| - Glycérine | | 5 g |
| - Ascorbate phosphate de magnésium | | 0,1 g |
| - Propylèneglycol | | 3 g |
| - Eau | | 40 g |
| - Diazolidinylurée vendue sous la dénomination "GERMAL II" par la Société SUTTON | | 0,1 g |
| - Mélange de méthylchloroisothiazolinone et de méthylisothiazolinone vendu sous la dénomination "KATHON CG" par la Société ROHM & HAAS | | 0,05 g |
| - Huile de silicone | | 1 g |
| - Acide polyacrylique réticulé vendu sous la dénomination "CARBOPOL 940" par la Société GOODRICH | | 0,25 g |
| - Paraoxybenzoate de méthyle | | 0,1 g |
| - Triéthanolamine | | 0,2 g |
| - Eau | qsp | 100 g |

EXEMPLE 5

**BAUME APRES-RASAGE**

On prépare la dispersion vésiculaire suivante :

| | |
|---|---|
| - Composé de l'exemple 1 | 1,9 g |
| - Cholestérol | 1,9 g |
| - Stéaroylglutamate monosodique vendu sous la dénomination "ACYL GLUTAMATE HS 11" par la Société AJINOMOTO | 0,2 g |
| - Allantoïne | 0,1 g |
| - Ethylmaltol | 0,4 g |
| - Polymère carboxyvinylique vendu sous la dénomination de "CARBOPOL 940" par la Société GOODRICH | 0,25 g |
| - Parahydroxybenzoate de méthyle | 0,1 g |
| - Diméthylol-1,3 diméthyl-5,5 hydantoïne vendu sous la nomination "GLYDANT" par la Société GLYCO | 0,055 g MA |
| - Eau déminéralisée | qsp 100 g |

EXEMPLE 6

**FORMULATION SOLAIRE**

On prépare la dispersion vésiculaire suivante :

| | |
|---|---|
| - Composé de l'exemple 1 | 3,8 g |
| - Cholestérol | 3,8 g |
| - Phosphate de dihexadécyle | 0,4 g |
| - Glycérine | 2 g |
| - Huile de vaseline | 15 g |
| - Paraméthoxycinnamate de 2-éthylhexyle | 5 g |
| - 2-hydroxy 4-méthoxybenzophénone | 2 g |
| - Polymère carboxyvinylique vendu sous la dénomination "CARBOPOL 940" par la Société GOODRICH | 0,4 g |
| - Triéthanolamine qs pH=6,5 | |
| - Conservateurs qs | |
| - Parfum qs | |
| - Eau déminéralisée | qsp 100 g |

EXEMPLE 7

**EMULSION HUILE-DANS-EAU**

*Phase A*

| | |
|---|---|
| - Polyisobutylène hydrogéné | 6,5 g |
| - Palmitate d'octyle | 5 g |
| - Huile de silicone volatile vendue sous la dénomination "VOLATIL SILICONE 7158" par la Société UNION CARBIDE | 5 g |
| - Alcool cétylique | 4 g |
| - Monostéarate de glycérol | 3 g |
| - Stéarate de polyéthylèneglycol à 40 moles d'oxyde d'éthylène, vendu sous la dénomination "MYRJ 52" par la Société I.C.I. | 2 g |
| - Myristate de myristyle | 2 g |
| - Tristéarate de sorbitan | 0,9 g |
| - Sel dipotassique de l'acide éthylènediamine tétracétique | 0,05 g |
| - Composé de l'exemple 1 | 1,5 g |

*Phase B*

| | |
|---|---|
| - Conservateur qs | |
| - Glycérine | 3 g |
| - Eau | 100 g |

On chauffe la phase A à 85°C. Parallèlement, on chauffe B à 95°C. On verse la phase B dans A et on émulsionne au MORITZ.

On obtient une crème blanche.

EXEMPLE 8

**EMULSION EAU-DANS-HUILE**

*Phase A*

| | |
|---|---|
| - Mélange de lanolate de magnésium et d'huile de vaseline (50/50) vendu sous la dénomination "MEXANYL GO" par la Société CHIMEX | 5,7 g |
| - Lanoline hydrogénée | 6,65 g |
| - 2-éthylhexyléther de palmitate de glycéryle | 2 g |

*Phase B*

| | |
|---|---|
| - Composé de l'exemple 1 | 3 g |
| - Mélange d'éthyl-2 hexanoate de cétostéaryle et de myristate d'isopropyle (90-10) vendu sous la dénomination "PURCELLIN LIQUIDE HUILE 2/066210" par la Société DRAGOGO | 3 g |
| - Mélange d'alcool de lanoline et d'huile de vaseline (15/85) vendu sous la dénomination "LIQUIDE BASE CB1145" par la Société CRODA | 3 g |
| - Palmitate d'isopropyle | 4,75g |
| - Huile de vaseline | 7,9 g |
| - Vaseline | 15 g |
| - Squalane | 0,4 g |
| - Mélange de mono-, di-, tri-glycérides d'acides oléique, linoléique, linolénique, et stéarique, vendu sous la dénomination "VITA COS" par la Société KOLMAR | 0,2 g |
| - Parfum | 0,8 g |

*Phase C*

| | |
|---|---|
| - Conservateur qs | |
| - Eau | qsp 100 g |

On chauffe le mélange A à 85°C. On fond B à 80°C.
On verse B dans A. On refroidit à 40°C.
On ajoute C à 40°C au mélange précédent. On refroidit trois fois à la tricylindre.
On obtient une crème blanche.

## Revendications

1. Composé de formule :

$$R_f-(CH_2)_m-S-G_n-H \qquad (I)$$

dans laquelle :
$R_f$ désigne un radical alkyle perfluoré, linéaire ou ramifié, en $C_6$ à $C_{20}$ ou un mélange de radicaux alkyle perfluorés, linéaires ou ramifiés, en $C_4$ à $C_{20}$;
m représente O, 1 ou 2;
n représente une valeur statistique ou entière comprise entre 2 et 10;
G représente un motif choisi parmi :

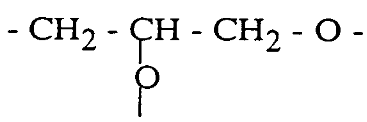 , 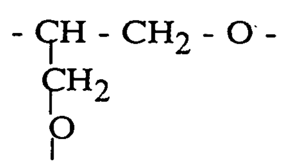 ,

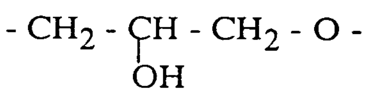 , 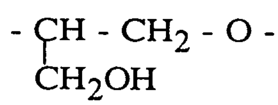 ,

ou

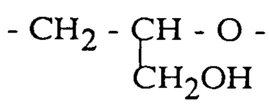 ,

chacun des atomes d'oxygène pouvant être relié à un atome d'hydrogène ou à un autre motif G.

2. Composé selon la revendication 1, caractérisé en ce que $R_f$ désigne un radical alkyle perfluoré, linéaire ou ramifié, en $C_6$ à $C_{14}$ et m est 2.

3. Composition cosmétique ou pharmaceutique, caractérisée en ce qu'elle comporte au moins un composé de formule :

$$R_f-(CH_2)_m-S-G_n-H \qquad (I)$$

dans laquelle :
$R_f$ désigne un radical alkyle perfluoré, linéaire ou ramifié, en $C_6$ à $C_{20}$ ou un mélange de radicaux alkyle perfluorés, linéaires ou ramifiés, en $C_4$ à $C_{20}$;
m représente O, 1 ou 2;
n représente une valeur statistique ou entière comprise entre 2 et 10;

G représente un motif choisi parmi :

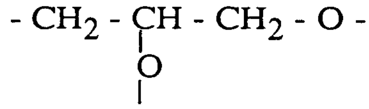 , 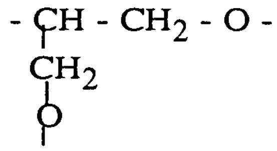 ,

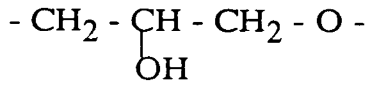 , 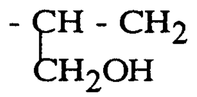 ,

ou

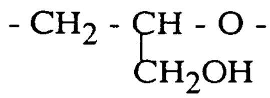 ,

chacun des atomes d'oxygène pouvant être relié à un atome d'hydrogène ou à un autre motif G.

4. Composition selon la revendication 3, caractérisée en ce que la composition se présente sous forme d'émulsions, de micro-émulsions, de laits ou de crèmes, de lotions, de gels, de bâtonnets solides, de pâtes, de sprays ou de mousses aérosols.

5. Composition selon l'une des revendications 3 ou 4, caractérisée en ce que les composés de formule (I) représentent 0,1 à 50% et de préférence 0,1 à 25% en poids du poids total de la composition.

6. Composition selon l'une des revendications 3 à 5, caractérisée en ce qu'elle comporte au moins un autre constituant choisi parmi les agents de surface ioniques ou non-ioniques, les polymères naturels ou synthétiques, ioniques ou non-ioniques, les huiles ou les cires, les protéines, les épaississants, les nacrants, les émollients, les hydratants, les colorants, les agents réducteurs ou oxydants, les conservateurs, les parfums, les filtres anti-UV, les solvants, les propulseurs et les produits actifs pharmaceutiques ou para-pharmaceutiques.

7. Composition selon l'une des revendications 3 à 6, caractérisée par le fait qu'elle se présente sous forme d'émulsion et qu'elle comporte, en outre, d'autres agents émulsionnants tels que des acides gras ou des alcools gras polyoxyéthylénés, des alkyléthers de polyglycérol, des esters d'acide gras et de sorbitan polyoxyéthylénés ou non, des esters d'acide gras et de sorbitol polyoxyéthylénés ou non, l'huile de ricin polyoxyéthylénée, des sels d'acides gras et d'amines ou de métaux polyvalents, des alkylsulfates polyoxyéthylénés ou non et des alkylphosphates polyoxyéthylénés ou non.

8. Composition cosmétique ou pharmaceutique comportant, dispersées dans une phase aqueuse de dispersion, des vésicules délimitées par un ou plusieurs feuillets formés d'une phase lipidique, contenant au moins un composé de formule (I) :

$$R_f-(CH_2)_m-S-G_n-H \qquad (I)$$

dans laquelle :
$R_f$ désigne un radical alkyle perfluoré, linéaire en $C_6$ à $C_{10}$;
m est 2;
n représente une valeur statistique ou entière comprise entre 2 et 3;

G représente un motif choisi parmi :

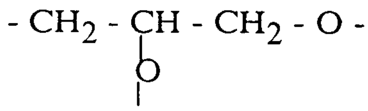 , 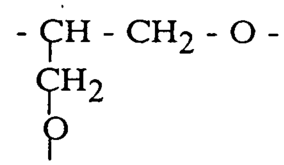 ,

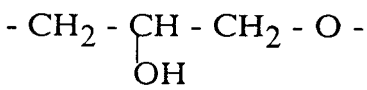 , 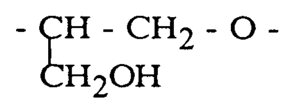 ,

ou

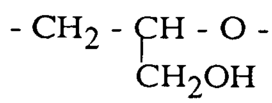 ,

chacun des atomes d'oxygène pouvant être relié à un atome d'hydrogène ou à un autre motif G.

9. Composition selon la revendication 4 ou 8, caractérisée en ce qu'elle comporte en outre un lipide amphiphile ionique ou non-ionique susceptible de former des vésicules stables, en mélange avec les composés de formule (I) pour constituer des membranes lipidiques de vésicules.

10. Composition selon l'une des revendications 8 ou 9, caractérisée par le fait que les vésicules contiennent au moins un actif cosmétique et/ou dermopharmaceutique dans la phase lipidique et/ou dans la phase encapsulée.

11. Composition selon l'une des revendications 8 à 10, caractérisée par le fait que la phase aqueuse de dispersion des vésicules contient au moins un actif cosmétique et/ou dermopharmaceutique hydrosoluble et/ou au moins un actif amphiphile.

12. Composition selon la revendication 10, caractérisée par le fait que les parois des vésicules contiennent au moins un actif cosmétique et/ou dermopharmaceutique liposoluble.

13. Composition selon l'une des revendications 6 à 12, caractérisée par le fait que la phase aqueuse de dispersion contient une dispersion de gouttelettes d'un liquide non miscible à l'eau.

14. Composition selon la revendication 13, caractérisée par le fait que le liquide non miscible à l'eau contient au moins un actif cosmétique et/ou dermopharmaceutique liposoluble.

15. Composition selon la revendication 13, caractérisée par le fait que le liquide non miscible à l'eau est choisi dans le groupe formé par les huiles animales ou végétales formées par des esters d'acide gras et de polyols, les huiles essentielles, naturelles ou synthétiques, les hydrocarbures halogénés, les silicones, les esters d'acide minéral et d'un alcool, les éthers et les polyéthers.

16. Composition selon l'une des revendications 6 à 15, caractérisée par le fait que l'actif cosmétique et/ou dermopharmaceutique est choisi dans le groupe formé par les anti-oxydants ou les anti-radicaux libres, les agents hydratants ou humectants, les agents mélanorégulateurs accélérateur de bronzage, les agents mélanorégulateurs dépigmentant, les agents de coloration de la peau, les liporégulateurs, les agents anti-vieillissements et anti-rides, les agents anti-UV, les agents kératolytiques, les émollients, les agents anti-inflammatoires, les agents rafraîchissants, les agents cicatrisants, les agents protecteurs vasculaires, les agents anti-bactériens, les agents antifongiques, les agents insectifuges, les agents antiperspirant, les agents déodorants, les agents anti-pelliculaires, les anti-chutes des cheveux, les colorants capillaires, les agents décolorants pour cheveux, les réducteurs pour permanente, les agents conditionneurs pour la peau et les cheveux.

**17.** Utilisation des composés de formule (I) :

$$R_f-(CH_2)_m-S-G_n-H \quad (I)$$

dans laquelle :
$R_f$ désigne un radical alkyle perfluoré, linéaire ou ramifié, en $C_6$ à $C_{20}$ ou un mélange de radicaux alkyle perfluorés, linéaires ou ramifiés, en $C_4$ à $C_{20}$;
m représente O, 1 ou 2;
n représente une valeur statistique ou entière comprise entre 2 et 10;
G représente un motif choisi parmi :

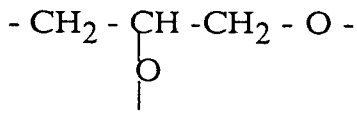 , 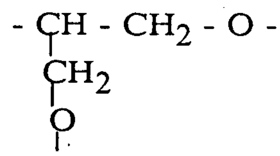 ,

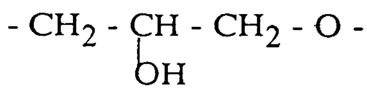 , 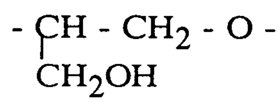 ,

ou

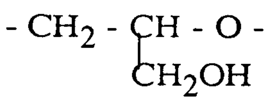 ,

chacun des atomes d'oxygène pouvant être relié à un atome d'hydrogène ou à un autre motif G, à titre d'agents tensio-actifs.

**18.** Vésicule, caractérisée en ce qu'elle est délimitée par un ou plusieurs feuillets formés d'une phase lipidique contenant au moins un composé de formule (I) :

$$R_f-(CH_2)_m-S-G_n-H \quad (I)$$

dans laquelle :
$R_f$ désigne un radical alkyle perfluoré, linéaire en $C_6$ à $C_{10}$ ;
m est 2;
n représente une valeur statistique ou entière comprise entre 2 et 3; et

G représente un motif

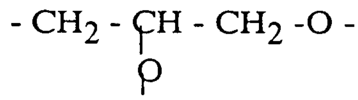 , 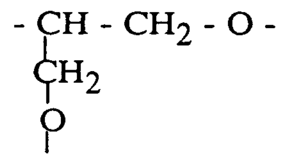 ,

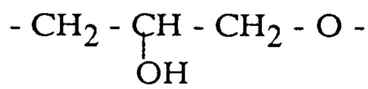 , 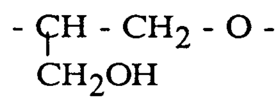 ,

ou

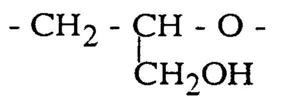 ,

chacun des atomes d'oxygène pouvant être relié à un atome d'hydrogène ou à un autre motif G.

**19.** Procédé de préparation d'un composé de formule (I) telle que définie à la revendication 1, caractérisé en ce qu'il comporte au moins la réaction d'un mercaptan fluoré de formule :

$$R_f\text{—}(CH_2)_m\text{—}S\text{—}H \qquad (II)$$

$R_f$ désignant un radical alkyle perfluoré, linéaire ou ramifié, en $C_6$ à $C_{20}$ ou un mélange de radicaux alkyle perfluorés, linéaires ou ramifiés, en $C_4$ à $C_{20}$;
m représentant O, 1 ou 2;
en présence d'une quantité active de catalyseur basique,

a) par condensation avec n moles

- de glycidol, ou
- d'un composé à fonction époxyde susceptible, après réaction, de régénéner une fonction alcool, éventuellement suivie d'une neutralisation;

ou
b) sur l'éther de glycidyle et d'isopropylidèneglycéryle, quand n, est une valeur entière égale à 2, ou à un multiple de 2 éventuellement suivie d'une hydrolyse;
ou
c) sur l'éther de glycidyle et de diisopropylidènetriglycéryle, quand n, est une valeur réelle, égale à 4,

éventuellement suivie d'une hydrolyse.

**Claims**

**1.** Compound of formula:

$$R_f\text{—}(CH_2)_m\text{—}S\text{—}G_n\text{—}H \qquad (I)$$

in which:

$R_f$ denotes a linear or branched $C_6$ to $C_{20}$ perfluoroalkyl radical or a mixture of linear or branched $C_4$ to $C_{20}$ perfluoroalkyl radicals;
m represents 0, 1 or 2;
n represents a statistical value or integer between 2 and 10;
G represents a unit chosen from:

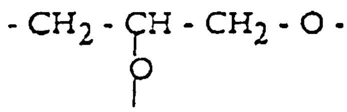 , 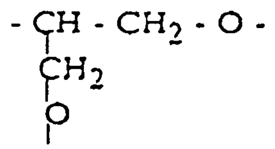 ,

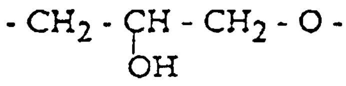 , 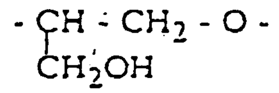 ,

or

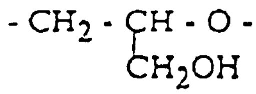 ,

it being possible for each of the oxygen atoms to be linked to a hydrogen atom or to another unit G.

**2.** Compound according to Claim 1, characterized in that $R_f$ denotes a linear or branced $C_6$ to $C_{14}$ perfluoroalkyl radical and m is 2.

**3.** Cosmetic or pharmaceutical composition, characterized in that it contains at least one compound of formula:

$$R_f-(CH_2)_m-S-G_n-H \qquad (I)$$

in which:
$R_f$ denotes a linear or branched $C_6$ to $C_{20}$ perfluoroalkyl radical or a mixture of linear or branched $C_4$ to $C_{20}$ perfluoroalkyl radicals;
m represents 0, 1 or 2;
n represents a statistical value or integer between 2 and 10;
G represents a unit chosen from:

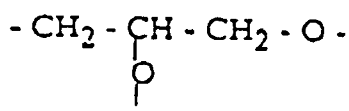 , 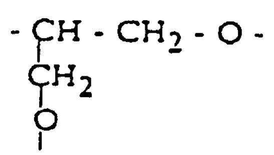 ,

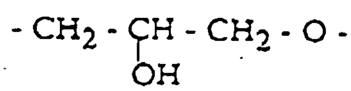 , 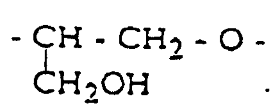 ,

or

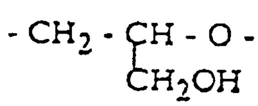 ,

it being possible for each of the oxygen atoms to be linked to a hydrogen atom or to another unit G.

4. Composition according to Claim 3, characterized in that the composition takes the form of emulsions, microemulsions, milks or creams, lotions, gels, solid sticks, pastes, sprays or aerosol foams.

5. Composition according to one of Claims 3 and 4, characterized in that the compounds of formula (I) represent 0.1 to 50%, and preferably 0.1 to 25%, by weight of the total weight of the composition.

6. Composition according to one of Claims 3 to 5, characterized in that it contains at least one other constituent chosen from ionic or nonionic surface-active agents, ionic or nonionic, natural or synthetic polymers, oils or waxes, proteins, thickeners, pearlescence agents, emollients, hydrating agents, colorants, reducing or oxidizing agents, preservatives, perfumes, anti-UV screening agents, solvents, propellants and pharmaceutical or parapharmaceutical active products.

7. Composition according to one of Claims 3 to 6, characterized in that it takes the form of an emulsion and in that it contains, in addition, other emulsifying agents such as polyoxyethylenated fatty acids or fatty alcohols, polyglycerol alkyl ethers, esters of fatty acid and of sorbitan, polyoxyethylenated or otherwise, esters of fatty acid and of sorbitol, polyoxyethylenated or otherwise, polyoxyethylenated castor oil, salts of fatty acids and of amines or of multivalent metals, alkyl sulphates, polyoxyethylenated or otherwise, and alkyl phosphates, polyoxyethylenated or otherwise.

8. Cosmetic or pharmaceutical composition containing, dispersed in an aqueous dispersion phase, vesicles bounded by one or more lamellae composed of a lipid phase containing at least one compound of formula (I):

$$R_f-(CH_2)_m-S-G_n-H \qquad (I)$$

in which:
$R_f$ denotes a linear $C_6$ to $C_{10}$ perfluoroalkyl radical,
m represents 2;
n represents a statistical value or integer between 2 and 3;
G represents a unit chosen from:

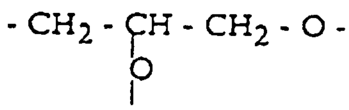

,

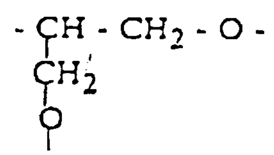

,

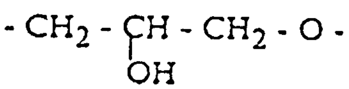

,

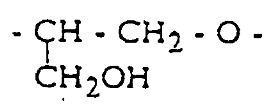

,

or

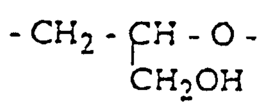

,

it being possible for each of the oxygen atoms to be linked to a hydrogen atom or to another unit G.

9. Composition according to Claim 4 or 8, characterized in that it contains, in addition, an ionic or nonionic amphiphilic lipid capable of forming stable vesicles, mixed with the compounds of formula (I) to constitute lipid membranes of vesicles.

10. Composition according to one of Claims 8 and 9, characterized in that the vesicles contain at least one cosmetic and/or dermopharmaceutical active compound in the lipid phase and/or in the encapsulated phase.

**11.** Composition according to one of Claims 8 to 10, characterized in that the aqueous phase of dispersion of the vesicles contains at least one water-soluble cosmetic and/or dermopharmaceutical active compound and/or at least one amphiphilic active compound.

**12.** Composition according to Claim 10, characterized in that the walls of the vesicles contain at least one fat-soluble cosmetic and/or dermopharmaceutical active compound.

**13.** Composition according to one of Claims 6 to 12, characterized in that the aqueous dispersion phase contains a dispersion of droplets of a water-immiscible liquid.

**14.** Composition according to Claim 13, characterized in that the water-immiscible liquid contains at least one fat-soluble cosmetic and/or dermopharmaceutical active compound.

**15.** Composition according to Claim 13, characterized in that the water-immiscible liquid is chosen from the group composed of animal or vegetable oils composed of esters of a fatty acid and of polyols, natural or synthetic essential oils, halogen-containing hydrocarbons, silicones, esters of an inorganic acid and of an alcohol, ethers and polyethers.

**16.** Composition according to one of Claims 6 to 15, characterized in that the cosmetic and/or dermopharmaceutical active compound is chosen from the group composed of antioxidants or anti-free radical agents, hydrating or humectant agents, melanoregulatory agents which accelerate tanning, depigmenting melanoregulatory agents, skin colouring agents, liporegulators, anti-aging and antiwrinkie agents, anti-UV agents, keratolytic agents, emollients, anti-inflammatory agents, refreshing agents, cicatrizing agents, vascular protective agents, antibacterial agents, antifungal agents, insect-repellant agents, antiperspirant agents, deodorant agents, anti-dandruff agents, agents for combating hair loss, hair colorants, hair bleaching agents, reducing agents for permanent-waving, and skin and hair conditioning agents.

**17.** Use of the compounds of formula (I):

$$R_f - (CH_2)_m - S - (G)_n - H \qquad (I)$$

in which:
$R_f$ denotes a linear or branched $C_6$ to $C_{20}$ perfluoroalkyl radical or a mixture of linear or branched $C_4$ to $C_{20}$ perfluoroalkyl radicals;
m represents 0, 1 or 2;
n represents a statistical value or integer between 2 and 10;
G represents a unit chosen from:

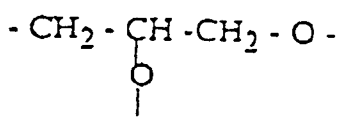 , 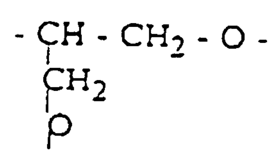 ,

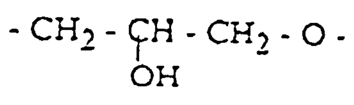 , 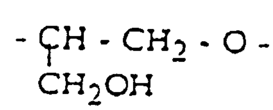 ,

or

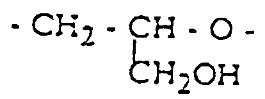 ,

it being possible for each of the oxygen atoms to be linked to a hydrogen atom or to another unit G, as surfactants.

**18.** Vesicle, characterized in that it is bounded by one or more lamellae composed of a lipid phase containing at least one compound of formula (I):

$$R_f-(CH_2)_m-S-G_n-H \qquad (I)$$

in which:
$R_f$ represents a linear $C_6$ to $C_{10}$ perfluoroalkyl radical,
m is equal to 2;
n represents a statistical value or integer between 2 and 3; and
G represents a unit

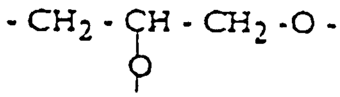

,

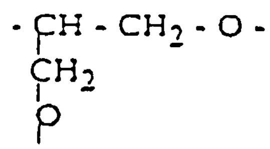

,

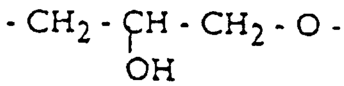

,

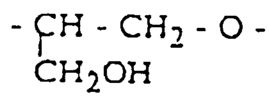

,

or

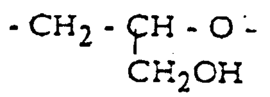

,

it being possible for each of the oxygen atoms to be linked to a hydrogen atom or to another unit G.

**19.** Process for preparing a compound of formula (I) as defined in Claim 1, characterized in that it entails at least the reaction of a fluorine-containing mercaptan of formula:

$$R_f-(CH_2)_m-S-H \qquad (II)$$

$R_f$ denoting a linear or branched $C_6$ to $C_{20}$ perfluoroalkyl radical or a mixture of linear or branched $C_4$ to $C_{20}$ perfluoroalkyl radicals;
m representing 0, 1 or 2;
in the presence of an active amount of basic catalyst,

a) by condensation with n mol

- of glycidol, or
- of a compound containing an epoxide function which is capable, after reaction, of regenerating an alcohol function,
  optionally followed by a neutralization;

or
b) with glycidyl isopropylideneglyceryl ether, when n, an integer, is equal to 2 or a multiple of 2,
optionally followed by a hydrolysis;
or

c) with glycidyl diisopropylidenetriglyceryl ether, when n is a integer equal to 4, optionally followed by a hydrolysis.

**Patentansprüche**

1. Verbindung der Formel:

$$R_f-(CH_2)_m-S-G_n-H \quad (I)$$

worin gilt:
$R_f$ bedeutet einen linearen oder verzweigten $C_{6-20}$-Perfluoralkylrest oder eine Mischung aus linearen oder verzweigten $C_{4-20}$-Perfluoralkylresten;
m stellt 0, 1 oder 2 dar;
n stellt einen statistischen oder ganzzahligen Wert von 2 bis 10 dar;
G stellt eine Einheit dar, die ausgewählt ist aus:

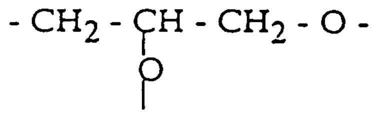
,
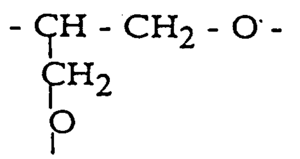
,

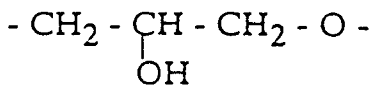
,
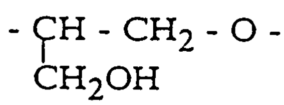
,

oder

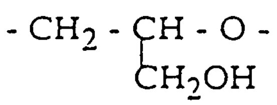
,

wobei jedes der Sauerstoffatome an ein Wasserstoffatom oder an eine weitere Einheit G gebunden ist.

2. Verbindung gemäß Anspruch 1,
dadurch **gekennzeichnet**, daß
$R_f$ einen linearen oder verzweigten $C_{6-14}$-Perfluoralkylrest bedeutet und m 2 ist.

3. Kosmetische oder pharmazeutische Zusammensetzung,
dadurch **gekennzeichnet**, daß
sie mindestens eine Verbindung der Formel enthält:

$$R_f-(CH_2)_m-S-G_n-H \quad (I)$$

worin gilt:
$R_f$ bedeutet einen linearen oder verzweigten $C_{6-20}$-Perfluoralkylrest oder eine Mischung aus linearen oder verzweigten $C_{4-20}$-Perfluoralkylresten;
m stellt 0, 1 oder 2 dar;
n stellt einen statistischen oder ganzzahligen Wert von 2 bis 10 dar;

G stellt eine Einheit dar, die ausgewählt ist aus:

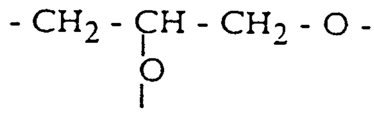 , 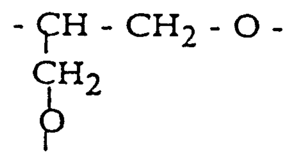 ,

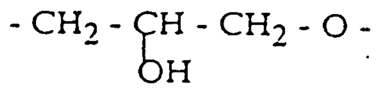 , 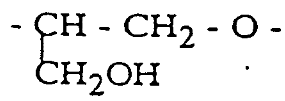 ,

oder

+ 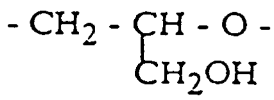 ,

wobei jedes der Sauerstoffatome an ein Wasserstoffatom oder an eine weitere Einheit G gebunden ist.

**4.** Zusammensetzung gemäß Anspruch 3, dadurch **gekennzeichnet**, daß die Zusammensetzung in Form von Emulsionen, Mikroemulsionen, Milch- oder Creme-Produkten, Lotionen, Gelen, Feststoffstäbchen, Pasten, Sprayprodukten oder Aerosol-Schäumen vorliegt.

**5.** Zusammensetzung gemäß einem der Ansprüche 3 oder 4, dadurch **gekennzeichnet**, daß die Verbindungen der Formel (I) 0,1 bis 50, vorzugsweise 0,1 bis 25, Gew.% des Gesamtgewichts der Zusammensetzung ausmachen.

**6.** Zusammensetzung gemäß einem der Ansprüche 3 bis 5, dadurch **gekennzeichnet**, daß sie mindestens einen weiteren Bestandteil enthält, ausgewählt aus ionischen oder nicht-ionischen Oberflächenmitteln, natürlichen oder synthetischen, ionischen oder nicht-ionischen Polymeren, Ölen oder Wachsen, Proteinen, Verdickungsmitteln, Perlmuttglanzmitteln, Weichmachern, Hydratisiermitteln, Färbemitteln, Reduktions- oder Oxidationsmitteln, Konservierungsstoffen, Parfüm-Produkten, Anti-UV-Filterstoffen, Lösungsmitteln, Treibmitteln und aus pharmazeutischen oder parapharmazeutischen Wirkstoffprodukten.

**7.** Zusammensetzung gemäß einem der Ansprüche 3 bis 6, dadurch **gekennzeichnet**, daß sie in Form einer Emulsion vorliegt und, zusätzlich, weitere Emulgiermittel wie polyoxethylierte Fettsäuren oder Fettlakohole, Polyglycerylalkylether, Sorbitanfettsäureester, polyoxethyliert oder nicht, Sorbitolfettsäureester, polyoxethyliert oder nicht, polyoxethyliertes Rizinusöl, Salze von Fettsäuren und von Aminen oder mehrwertigen Metallen, gegebenenfalls polyoxethylierte Alkylsulfate und Alkylphosphate enthält.

**8.** Kosmetische oder pharmazeutische Zuammensetzung, enthaltend, dispergiert in einer wässrigen Dispersionsphase, Bläschen, die durch ein oder mehrere Blättchen umgrenzt sind, welche aus einer lipidischen Phase gebildet sind, die mindestens eine Verbindung der Formel (I) enthält:

$$R_f — (CH_2)_m — S — G_n — H \qquad (I)$$

worin gilt:

$R_f$ bedeutet einen linearen oder verzweigten $C_{6-20}$-Perfluoralkylrest oder eine Mischung aus linearen oder verzweigten $C_{4-20}$-Perfluoralkylresten;
m stellt 0, 1 oder 2 dar;
n stellt einen statistischen oder ganzzahligen Wert von 2 bis 10 dar;
G stellt eine Einheit dar, die ausgewählt ist aus:

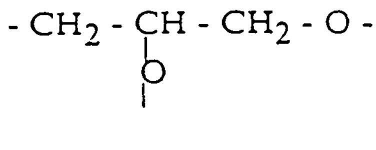 , 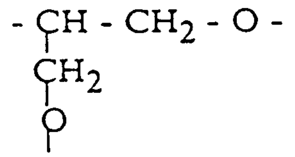 ,

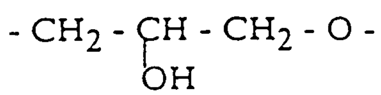 , 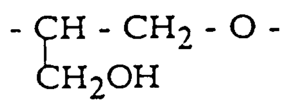 ,

oder

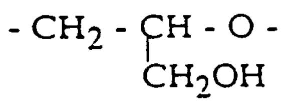 ,

wobei jedes der Sauerstoffatome an ein Wasserstoffatom oder an eine weitere Einheit G gebunden ist.

**9.** Zusammensetzung gemäß Anspruch 4 oder 8,
dadurch **gekennzeichnet**, daß
sie ausserdem ein ionisches oder nicht-ionisches amphiphiles Lipid zur Bildung stabiler Bläschen gemischt mit den Verbindungen der Formel (I) enthält, um lipidische Membranen aus Bläschen aufzubauen.

**10.** Zusammensetzung gemäß einem der Ansprüche 8 oder 9,
dadurch **gekennzeichnet**, daß
die Bläschen mindestens einen kosmetischen und/oder dermopharamzeutischen Wirkstoff in der lipidischen und/oder eingekapselten Phase enthalten.

**11.** Zusammensetzung gemäß einem der Ansprüche 8 bis 10,
dadurch **gekennzeichnet**, daß
die wässrige Dispersionsphase der Bläschen mindestens einen kosmetischen und/oder dermopharmazeutischen wasserlöslichen und/oder mindestens einen amphiphilen Wirkstoff enthält.

**12.** Zusammensetzung gemäß Anspruch 10,
dadurch **gekennzeichnet**, daß
die Wände der Bläschen mindestens einen kosmetischen und/oder dermopharmazeutischen fettlöslichen Wirkstoff enthalten.

**13.** Zusammensetzung gemäß einem der Ansprüche 6 bis 12,
dadurch **gekennzeichnet**, daß
die wässrige Phase der Dispersion eine Dispersion aus Tröpfchen einer mit Wasser nicht mischbaren Flüssigkeit enthält.

**14.** Zusammensetzung gemäß Anspruch 13,
dadurch **gekennzeichnet**, daß

die mit Wasser nicht mischbare Flüssigkeit mindestens einen kosmetischen und/oder dermopharmazeutischen fettlöslichen Wirkstoff enthält.

**15.** Zusammensetzung gemäß Anspruch 13,
dadurch **gekennzeichnet**, daß
die mit Wasser nicht mischbare Flüssigkeit aus der Gruppe aus tierischen oder pflanzlichen Ölen aus Estern einer Fettsäure und von Polyolen, aus natürlichen oder synthetischen essentiellen Ölen, Halogenkohlenwasserstoffen, Silikonen, Estern einer Mineralsäure und eines Alkohols, Ethern und aus Polyethern ausgewählt ist.

**16.** Zusammensetzung gemäß einem der Ansprüche 6 bis 15,
dadurch **gekennzeichnet**, daß
das kosmetische und/oder dermopharmazeutische Wirkstoffmittel aus der Gruppe aus Antioxidantien oder Abfangmitteln für freie Radikale, Hydratisier- oder Feuchtigkeitsmitteln, die Bräunung beschleunigenden Melanoregulatoren, entpigmentierenen Melanoregulatoren, Färbemitteln der Haut, Liporegulatoren, Mitteln gegen Alterung und Falten, Anti-UV-Mitteln, keratolytischen Mitteln, Weichmachern, entzündungshemmenden Mitteln, Erfrischungsmitteln, Vernarbungsmitteln, Gefäßschutzmitteln, antibakteriellen, fungiziden und insektiziden Mitteln, Antischweißmitteln, Deodoriermitteln, Antischuppenmitteln, Mitteln gegen Haarausfall, Haarfärbemitteln, Haarentfärbungsmitteln, Reduktionsmitteln für eine Dauerwelle sowie aus Konditioniermitteln für Haut und Haare ausgewählt ist.

**17.** Verwendung von Verbindungen der Formel (I):

$$R_f-(CH_2)_m-S-G_n-H \qquad (I)$$

worin
worin gilt:
$R_f$ bedeutet einen linearen oder verzweigten $C_{6-20}$-Perfluoralkylrest oder eine Mischung aus linearen oder verzweigten $C_{4-20}$-Perfluoralkylresten:
m stellt 0, 1 oder 2 dar;
n stellt einen statistischen oder ganzzahligen Wert von 2 bis 10 dar;
G stellt eine Einheit dar, die ausgewählt ist aus:

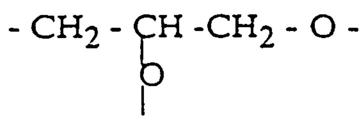
,
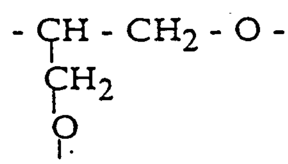
,

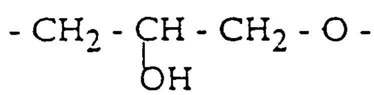
,
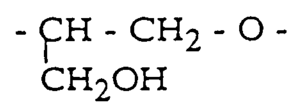
,

oder

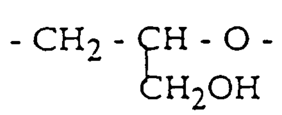
,

wobei jedes der Sauerstoffatome an ein Wasserstoffatom oder an eine weitere Einheit G gebunden ist, als oberflächenaktive Mittel.

**18.** Bläschen,
dadurch **gekennzeichnet**, daß

es von einem oder mehreren Blättchen umgrenzt ist, die aus einer lipidischen Phase gebildet sind, die mindestens eine Verbindung der Formel (I) enthält:

$$R_f-(CH_2)_m-S-G_n-H \quad (I)$$

worin gilt:
$R_f$ bedeutet einen linearen oder verzweigten $C_{6-20}$-Perfluoralkylrest oder eine Mischung aus linearen oder verzweigten $C_{4-20}$-Perfluoralkylresten;
m stellt 0, 1 oder 2 dar;
n stellt einen statistischen oder ganzzahligen Wert von 2 bis 10 dar;
G stellt eine Einheit dar, die ausgewählt ist aus:

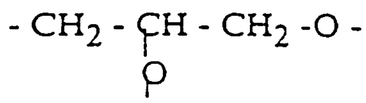 , 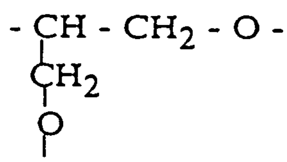 ,

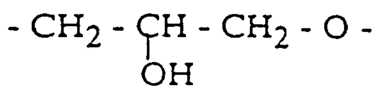 , 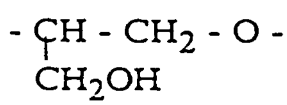 ,

oder

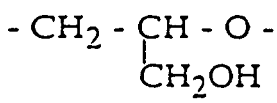 ,

wobei jedes der Sauerstoffatome an ein Wasserstoffatom oder an eine weitere Einheit G gebunden ist,

**19.** Verfahren zur Herstellung einer in Anspruch 1 definierten Verbindung der Formel (I),
dadurch **gekennzeichnet**, daß
man mindestens eine Kondensationsreaktion mit einem fluorhaltigen Mercaptan der Formel:

$$R_f-(CH_2)_m-S-H \quad (II)$$

worin $R_f$ einen linearen oder verzweigten $C_{6-20}$-Perfluoralkylrest oder eine Mischung aus linearen oder verzweigten $C_{4-20}$-Perfluoralkylresten bedeutet und m 0, 1 oder 2 darstellt,
in Gegenwart einer wirksamen Menge eines basischen Katalysators durchführt:

a) mit n Mol

- Glycidol oder
- einer Verbindung mit Epoxid-Funktion unter nach der Reaktion erfolgender Wiederbildung einer Alkohol-Funktion,

worauf gegebenenfalls neutralisiert wird;
oder

b) mit Glycidylisopropylidenglycerylether, wenn n ein ganzzahliger Wert von 2 oder einem Vielfachen von 2 ist, worauf gegebenenfalls hydrolysiert wird;
oder

c) mit Glycidyldiisopropylidentriglycerylether, wenn n ein reeller Wert von 4 ist,

worauf gegebenenfalls hydrolysiert wird.